# EUROPÄISCHE PATENTSCHRIFT

(11) **EP 1 216 720 B1**
(45) Veröffentlichungstag und Bekanntmachung des Hinweises auf die Patenterteilung: **09.06.2004**
(21) Anmeldenummer: 01128873.5
(22) Anmeldetag: 05.12.2001
(51) Int. Cl.: A61M 15/00

(54) **Vorrichtung zum Erfassen der Betätigung eines Spenders**
Means for registering the operation of an inhaler
Moyen pour enregistrer l'actionnement d'un inhalateur

(30) Priorität: 23.12.2000 DE 10065160
(43) Veröffentlichungstag der Anmeldung: 26.06.2002
(73) Patentinhaber: ING. ERICH PFEIFFER GMBH, 78315 Radolfzell (DE)
(72) Erfinder: Fuchs, Karl-Heinz, 78315 Radolfzell (DE)
(74) Vertreter: Patentanwälte , Ruff, Wilhelm, Beier, Dauster & Partner

(56) Entgegenhaltungen:
- EP-A- 0 448 204
- WO-A-00/64517
- WO-A-97/20589
- US-A- 5 544 647
- US-A- 5 809 997

## Beschreibung

Die Erfindung betrifft eine Vorrichtung zum Erfassen der Betätigung eines Spenders, insbesondere eines Spenders für feste oder flüssige vorzugsweise wenigstens einen pharmazeutischen Wirkstoff enthaltende Medien sowie eine Vorrichtung zum Austragen von derartigen Medien.

Dabei ist es beispielsweise aus der DE 33 02 160 A1 bekannt, bei einer Dosiereinrichtung bei der in einem Medienbehälter ein Medium bevorratet ist, das Medium mit einem Austragförderer aus dem Medienbehälter herauszufördern. Dabei erfolgt die Betätigung des Austragförderers durch eine Relativbewegung zwischen einem Betätigungselement und dem Medienbehälter. Darüber hinaus ist es aus dieser Druckschrift noch bekannt, die Anzahl der erfolgten Betätigungen dadurch zu erfassen, daß mechanisch bei jedem Betätigungshub ein Zählrad weitergedreht wird und somit nach jedem Hub eine andere beschriftete Stelle an einem Anzeigebereich oder Zeigerelement vorbeigeführt wird und somit die Anzahl der erfolgten Betätigungen repräsentiert. Dabei ist das Zählrad auf Seiten des Medienbehälters oder an einem den Medienbehälter umgebenen Gehäuse angeordnet. An dem Betätigungselement ausgebildete mechanische Schleppmittel sorgen dafür, daß bei einer Betätigung des Betätigungsmittels - einer Hubbewegung - ein Weiterdrehen des Zählringes in der vorgegebenen Drehrichtung erfolgt. Demgemäß ist bei einer solchen Zähleinrichtung das Zählelement auf der Seite des Medienbehälters angeordnet.

Dabei ist die Verwendung von Dosiereinrichtungen oder Spendern zum Austragen eines fließfähigen Stoffes oder eines Pulvers in Verbindung mit einer Zähleinrichtung insbesondere dann von Interesse, wenn das ausgetragene Medium einen pharmazeutischen Wirkstoff enthält. Besonders dann, wenn der pharmazeutische Wirkstoff hochwirksam und sehr genau dosiert werden muß, muß dem Benutzer auch die Möglichkeit gegeben werden, zu erfassen, wieviel Betätigungen bereits erfolgt sind, damit nicht unabsichtlich zuviel oder zuwenig Medikament verabreicht wird. Der Austrag aus solchen Spendern erfolgt meistens in Form eines Zerstäubens. Hierzu weisen derartige Spender beispielsweise im Mund/Rachenraum und in der Nase applizierbare Austragöffnungen auf.

Darüber hinaus können mit einem Zählrad oder anderen mechanischen Mitteln zwei- oder dreistellige Zahlenwerke nur schlecht dargestellt werden, da die verfügbare Wegstrecke zwischen zwei Skalenteilern extrem gering wird.

Die US 5 544 647 A sowie die EP 0 448 204 A1 beschreiben Inhataforen, die mit einer einem unter Druck stehenden Speicher für ein Medikament arbeiten. Dabei ist der Sprühkopf, der in ein Inhalatormundstück sprüht, in einem winkelförmigen Gehäuse angeordnet, in das der Druckspeicher von oben her eingesetzt ist. Durch Druck auf seinen Boden wird der Sprühvorgang ausgelöst. Dabei betätigt der Behälter einen Schalter, der eine elektrische Zählvorrichtung mit einer Anzeige, teilweise einer elektrischen Digitalanzeige, betätigt.

Vergleichbares zeigt auch die US 5 809 997 A.

Die WO 97/20589 beschreibt einen Inhalator mit einer Schutzkappe, die das Mundstück abdeckt und vor der Betätigung abgezogen wird, jedoch über zwei Laschen schwenkbar mit dem lnhalatorgehäuse verbunden bleibt. Beim Abziehen der Schutzkappe können mechanische Rüttelvorgänge ausgelöst werden oder dieser Vorgang kann zur Energiegewinnung für die Ladung eines Akkumulators benutzt werden.

Demgegenüber ist es Aufgabe der Erfindung, die Erfassungseinrichtung vor Fehlbetätigung und/oder unnötigen Energieverbrauch zu schützen. Vorteilhaft sollte auch die Erfassung von Betätigungen zum einen vereinfacht und mit einer geringeren Anzahl an Präzisions-Bauteilen durchzuführen und zum anderen sicherzustellen sein, daß nur vollständige Betätigungen des Betätigungselements erfasst werden. Darüber hinaus sollte auch noch eine größere Sicherheit vor Manipulationen gewährleistet und eine bessere Darstellbarkeit großer Zahlenwerte ermöglicht werden.

Diese Aufgabe wird durch die Merkmale des Anspruchs 1 gelöst.

Eine erfindungsgemäße Vorrichtung zum Erfassen der Betätigungen eines Spenders dient insbesondere zum Erfassen der Betätigung eines Spenders für feste oder flüssige, vorzugsweise wenigstens einen pharmazeutischen Wirkstoff (z.B. Opiate, Calcitonin, Oxitocin und freundsche Adjuvantien) enthaltende Medien. Dabei ist das Medium in einem Medienbehälter bevorratet. An dem Medienbehälter ist ein Austragförderer vorgesehen, die Betätigung des Austragförderers dient dem Austrag von Medium. Dabei erfolgt die Betätigung des Austragförderers durch eine Relativbewegung zwischen einem Betätigungselement und dem Medienbehälter. Gemäß der Erfindung ist im Betätigungselement eine Erfassungseinrichtung, beispielsweise ein Sensor, zur Erfassung erfolgter Betätigungen des Betätigungselements angeordnet. Die Erfassungseinrichtung erzeugt ein Zählsignal. Dieses Zählsignal dient der Erfassung erfolgter Betätigungen.

Gemäß einer bevorzugten Ausgestaltung der Erfindung wird das elektrische Zählsignal in einer elektrischen Zähleinrichtung verarbeitet, die vorzugsweise ebenfalls im Betätigungselement angeordnet ist.

Zum Anzeigen des in der Zähleinrichtung in Abhängigkeit der Anzahl erfolgter Zählsignale ermittelten Zählwertes dient ein im Betätigungselement angeordnetes Anzeigeelement, insbesondere ein elektrooptisches Anzeigeelement oder aber eine ein- oder mehrstellige 8-Segment-Anzeige. Ein Beispiel für ein derartiges elektrooptisches Anzeigeelement ist eine LCD-Anzeige. Es kann sich dabei auch um einen entsprechend kleinen Bildschirm, beispielsweise einen Plasmabildschirm handeln. Derartige elektrooptische Anzeigeelemente haben den Vorteil, daß eine nahezu beliebige Informationsdarstellung möglich ist. Es können nicht nur die Anzahl der noch verfügbaren oder der erfolgten Betätigungen als Zählwert angegeben werden, es können auch noch weitere Informationen angezeigt werden, z.B. die Anzahl der Betätigungen pro Zeiteinheit oder etwa wie lange seit der letzten Betätigung verstrichen ist oder wieviel Zeit vor der nächsten Betätigung mindestens noch verstreichen sollte. Diese Informationen können nicht nur als Zahlenwerte angezeigt werden, sondern auch piktogrammartig oder mit anderen Symbolen. Die Möglichkeit und die Anzahl der Anzeigen steigt dabei mit größer werdender Fläche des Anzeigeelements. Das Anzeigeelement ist gemäß der Erfindung im Betätigungselement angeordnet und soweit das Betätigungselement zwar in der Vertikalen gradlinig ausgebildet ist aber in der Querrichtung dazu gewölbt ist, kann auch eine entsprechende Wölbung des Anzeigeelements vorgesehen sein.

Eine besonders vorteilhafte Ausgestaltung ist gegeben, wenn Anzeigeelement und elektrische Zähleinrichtung als zusammenhängendes und einstückiges Bauteil ausgebildet sind. Dieses Bauteil enthält somit nicht nur die Steuerung der Anzeige und das physikalisch ausgebildete Anzeigeelement sondern auch die elektrische Zähleinrichtung, also die Auswertelogik für die übermittelten Zählsignale. Das zusammenhängende Bauteil ist dabei insbesondere die Steuereinheit der LCD-Anzeige.

Gemäß einer erfindungsgemäßen Vorrichtung zum Austragen von insbesondere festen oder flüssigen, vorzugsweise einen pharmazeutischen Wirkstoff enthaltenden Medien - beispielsweise einem Spender - weist einen Behälter zur Bevorratung austragbarem Mediums auf. Ein Betätigungselement wirkt derart auf einen Austragförderer ein, daß durch eine Betätigung des Betätigungselements ein Austrag von Medium aus dem Medienbehälter erzeugt wird. Gemäß der Erfindung ist in dem Betätigungselement ein elektrooptisches Anzeigeelement angeordnet.

Gemäß bevorzugter Ausgestaltung der Erfindung ist das Anzeigeelement als LCD-Anzeige ausgebildet. Gemäß weiterführender Ausgestaltung der Erfindung ist das Anzeigeelement an eine Auswerteeinheit angeschlossen, bzw. als Teil einer Auswerteeinheit ausgebildet, wobei in der Auswerteeinheit Signale eines Sensors verarbeitet werden und wobei die Anzeige Informationen in Abhängigkeit der Sensorsignale bestimmt. Dabei ist es vorteilhaft, wenn in dem Betätigungselement auch ein elektrischer Energiespeicher zur Energieversorgung des Anzeigeelements der Auswerteeinheit angeordnet ist.

Gemäß vorteilhafter Ausgestaltung der erfindungsgemäßen Vorrichtungen ist die Anzeigedauer des Anzeigeelements lediglich für eine bestimmte Anzeigedauer, die beispielsweise fest vorgegeben ist oder situationsabhängig bestimmt wird, aktiviert. Diese Begrenzung der Anzeigedauer dient in erster Linie der Limitierung des Energieverbrauches des Anzeigeelements und somit der Verlängerung der Haltbarkeit des Gerätes. Die Anzeigedauer ist dabei gemäß weiterführender Ausgestaltung der Erfindung vorzugsweise auf einen vorgegebenen Zeitraum nach dem letzten, die angezeigte Information verändernden Signal begrenzt. Ein die Anzeige veränderndes Signal ist beispielsweise ein Signal, daß in dem Sensor erfaßt wird und zur Veränderung des Zählwertes und damit auch zu Veränderung der in Abhängigkeit des Zählwertes angezeigten Information.

Dabei ist es möglich, daß das Anzeigeelement aus dem nicht aktivierten Nicht-Anzeigezustand in den Zustand versetzt werden kann, bei dem eine Anzeige von Informationen erfolgt, in dem eine ganz leichte Betätigung des Betätigungselements durchgeführt wird, die jedoch noch nicht zu einem Austrag von Medium führt. Vielen Spendern ist es gemeinsam, daß ein gewisser Leerweg des Betätigungsmittels überwunden werden muß, bevor ein Austragvorgang eingeleitet wird oder beginnt. Dieser Leerweg kann hierzu genutzt werden, in dem während des Leerweges ein Signal generiert wird, beispielsweise durch Kontaktierung oder Unterbrechung der Kontaktierung eines elektrischen Schalters, der mit der Auswerteeinheit verbunden ist.

Alternativ oder zusätzlich kann auch über eine geeignete Erfassungseinrichtung, beispielsweise über einen weiteren Schalter, erfaßt werden, ob eine Schutzkappe zum Schutz des Spenders bei Nichtgebrauch auf den Spender aufgesetzt ist oder nicht. Dabei kann über diese Erfassungseinrichtung sowohl das Abnehmen als auch das Aufsetzen der Schutzkappe erfaßt sein und alternativ oder sowohl als auch das Anordnen der Schutzkappe auf dem Spender ein Deaktivieren des Anzeigeelements und/oder das Entfernen der Schutzkappe von dem Spender ein Aktivieren des Anzeigeelements zur Folge haben.

Gemäß bevorzugter Ausgestaltungen der Erfindung ist das Betätigungselement als einstückiges, die Austragöffnung des Spenders beinhaltendes Bauteil ausgebildet. Dabei ist das Bauteil insbesondere gleichzeitig auch als am Verabreichungsort anzusetzender Applikator insbesondere als nasaler oder bukaler Applikator ausgebildet.

Die vorstehenden und weiteren Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein können.

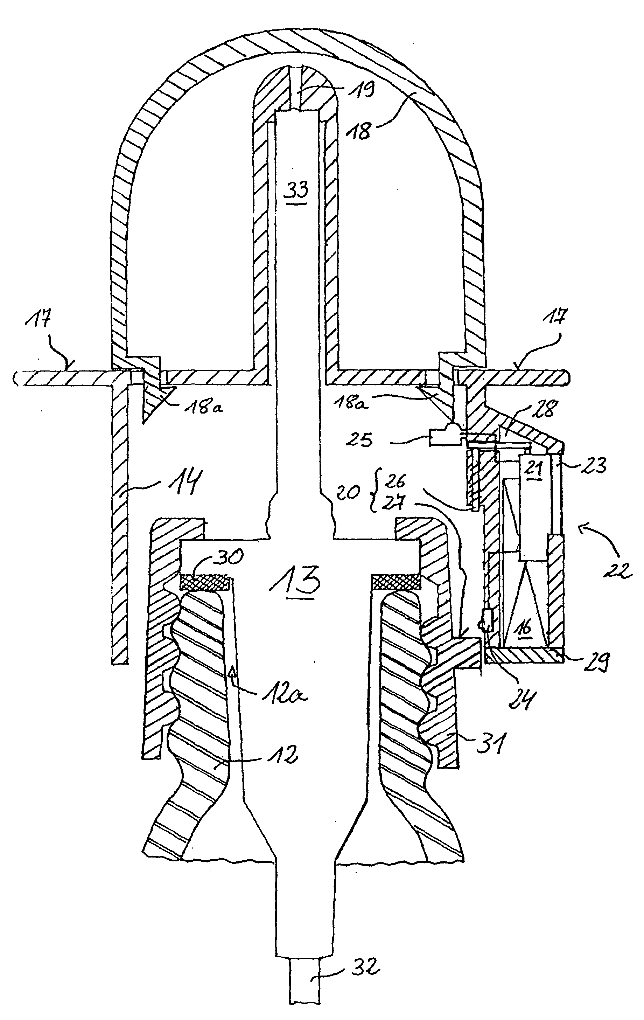

Diese und weitere Ausgestaltungen der Erfindung können auch den in der Zeichnung dargestellten Ausführungsbeispiel entnommen werden; die einzige Figur zeigt dabei:
eine teilgeschnittene schematische Darstellung erfindungsgemäßer Vorrichtungen.

Die Figur zeigt einen Spender 11 in seinem nur teilweise dargestellten Medienbehälter 12. In dem Medienbehälter 12 kann ein auszutragendes Medium bevorratet werden. Bei den auszutragenden Medien handelt es sich dabei entweder um Flüssigkeiten, die unterschiedliche Zähigkeiten aufweisen können und einem Bereich von hochviskosen Flüssigkeiten bis hin zu sehr dünnflüssigen Flüssigkeiten wie z.B. Lösungen auf Alkoholbasis beinhalten können. Darüber hinaus enthalten die bevorratenden Medien meist einen pharmazeutischen Wirkstoff.

In die Öffnung 12a des Medienbehälters ist der Austragförderer 13 eingesetzt. Der Austragförderer 13, im hier gezeichneten Ausführungsbeispiel handelt es sich um eine Tauchkolbenpumpe, ist mittels des Dichtrings 30 dichtend auf die Öffnung 12a (beispielsweise einer Zerstäuber-Düse) aufgesetzt und wird mittels der Halterungsschraube 31 fest am Medienbehälter 12 gehalten. Der Austragförderer 13 kann dabei beispielsweise eine Tauchkolbenpumpe sein, wie sie in dem dargestellten Ausführungsbeispiel gezeigt ist, die mit einem Saugschlauch 32 von einem möglichst tiefen Punkt des Medienbehälters 12 Medium ansaugt und das Medium durch das Steigrohr 33 hindurch bis zur Austragöffnung fördert.

Die Betätigung des Austragförderers 13 erfolgt durch eine Betätigung des Betätigungselements 14. Hierzu sind an dem Betätigungselement 14 Anlageflächen 17 ausgebildet, auf die der Benutzer seine Finger auflegen kann und dann durch ein Herunterdrücken des Betätigungselements 14 in Richtung auf den Medienbehälter 12 einen Austraghub der Tauchkolbenpumpe erzeugen kann. Zum Erzeugen der entsprechenden Gegenkraft kann beispielsweise der Daumen an der Unterseite des Medienbehälters angreifen. Bei jedem Austragshub erfolgt durch die Austragöffnung 19 hindurch ein Austrag von Medium. Hierzu ist das Betätigungselement 14 so ausgebildet, daß es über das Steigrohr 33 auf den Tauchkolben, der als Austragförderer 13 dienenden Tauchkolbenpumpe einwirkt. Das Betätigungselement kann um den Hubweg des Tauchkolbens relativ zum Austragförderer und somit relativ zum Medienbehälter verfahren werden. Um eine sichere Relativlage zwischen Betätigungselement 14 und Medienbehälter 12 herzustellen, ist es weiterhin auch möglich eine radiale Führung zwischen Halterungsschraube 31 oder Medienbehälter 12 und Betätigungselement 14 vorzusehen. Dies kann insbesondere dadurch geschaffen werden, daß die Halterungsschraube 31 eine nicht runde Außenkontur aufweist und die Form des Betätigungselements 14 an diese Form entsprechend angepaßt ist. Es können aber auch Führungsschienen, Führungsnuten und Leitflächen vorgesehen sein. Dabei ist es auch möglich, daß die Halterungsschraube 31 entweder ein Teil eines den Medienbehälter 12 umgebenden Gehäuses ist oder aber als Teil eines solchen ausgeformt ist.

Zum Schutz der Austragöffnung 19 vor Verschmutzung bei Nichtgebrauch des Spenders 11 aber auch um eventuell eine Sicherung des Spenders vor unerwünschter Betätigung bei Nichtgebrauch sicherzustellen, kann eine Schutzkappe 18 auf das Betätigungselement 14 aufgesetzt werden, daß die Austragöffnung 19 abdeckt.

Das dargestellte Betätigungselement weist eine Erfassungseinrichtung 20 auf, die dazu dient, erfolgte Betätigungen des Betätigungselements 14 zu signalisieren. Die Erfassungseinrichtung 20 besteht dabei aus dem Schaltelement 26, das auf Seiten des Betätigungselements 14 angeordnet ist, und der halterungsschraubenseitig ausgeformten Schaltfläche 27. Sobald das Schaltelement 26 in Anlage mit der Schaltfläche 27 gelangt, wird ein Zählsignal erzeugt, daß an die Zähleinrichtung 21 übermittelt wird. An die Zähleinrichtung 21 wird in Abhängigkeit der Anzahl erfaßter Zählsignale ein Zählwert ermittelt. Dabei kann es sich bei dem Zählwert in einfachster Ausführungsform um die Anzahl der insgesamt erfolgten Betätigungen des Betätigungselements handeln, es kann sich aber auch um die Anzahl der erfolgten Betätigungen innerhalb einer bestimmten Zeit, beispielsweise innerhalb einer Stunde oder innerhalb eines Tages, handeln. Alternativ oder ergänzend kann als Zählwert auch die Anzahl der noch verbleibenden Betätigungen angezeigt werden, die zu einem Austrag von Medium führen. Dies ist beispielsweise dann von Interesse, wenn in dem Medienbehälter eine genau dosierte Menge Medium vorhanden ist und der Nutzer darauf angewiesen ist, zu wissen, wie oft noch eine Dosis verabreicht werden kann, bevor der Behälter vollständig entleert ist. Die Erfassungseinrichtung 20 mit ihrem Schaltelement 26 und der Schaltfläche 27 bildet also einen Sensor für das Erfolgen einer vollständigen Betätigung des Betätigungselements 14.

Daneben ist an dem Betätigungselement 14 auch noch ein Leerwegschalter 24 angeordnet, der eine Betätigung des Betätigungsmittels 14 im Bereich des Leerweges, also ohne Durchführung eines Medienaustrages, erfaßt. Dieser Leerwegschalter 24, es kann sich dabei um einen Tastschalter handeln, übermittelt ein entsprechendes Schaltsignal zu Beginn jeglicher Betätigung des Betätigungselements 14. Aufgrund eines Schaltsignals des Leerwegschalter 24 kann das Anzeigeelement 22 angesteuert, insbesondere eingeschaltet werden.

Ferner ist ein Schutzkappenschalter 25, bei dem es sich ebenfalls um einen einfachen Tastschalter handeln kann, außerhalb des Verfahrweges des Behälters 12 und der Befestigungsschraube 31 am Betätigungselement 14 angeordnet. Über den Schutzkappenschalter 25 kann erfaßt werden, ob die Schutzkappe 25 auf die Austragvorrichtung 11, hier insbesondere auf das Betätigungselement 14 aufgesetzt ist. Zur Betätigung des Schutzkappenschalters 25 kann dabei insbesondere eine in das Innere des Betätigungselements 14 hineinragende Rastnase 18a der Schutzkappe 18 dienen, die zum Halten der Schutzkappe 18 auf dem Betätigungselement 14 dient. Der Schaltzustand des Schutzkappenschalters repräsentiert dann die beiden Zustände "Schutzkappe aufgesetzt" und "Schutzkappe nicht aufgesetzt". Ein entsprechendes Signal wird dem Anzeigeelement 22 zugeführt.

Das Anzeigelement 22 kann insbesondere aus einer LCD-Anzeige 23, einem kleinen Farb-Bildschirm und einer Ansteuerungselektronik bestehen. Die Ansteuerungselektronik und die Zähleinrichtung 21 sind dabei vorzugsweise als ein gemeinsames Elektronikbauteil ausgeführt. Auch die LCD-Anzeige 23 kann direkt auf diesem Elektronikbauteil aufgebracht sein. Die Energieversorgung des Anzeigelements und der Zähleinrichtung 21 erfolgt dabei durch den Energiespeicher 16, beispielsweise eine Batterie oder Knopfzelle. Zur Aufnahme von Zähleinrichtung 21, der Anzeigesteuerung und dem Anzeigeelement 22 ist hierzu an dem Betätigungselement 14 eine Kammer 28 ausgebildet, die mittels des Deckels 29 verschlossen wird. Dabei bildet das Anzeigeelement 22, beispielsweise der LCD-Bildschirm, den entsprechenden Abschluß einer Öffnung in der Kammer 28 hin zum Benutzer oder Betrachter. Der Deckel 29 kann dabei sowohl lösbar als auch unlösbar dichtend oder auch nicht hermetisch abschließend am Betätigungselement 14 angeordnet sein.

Nachfolgend ist noch die Arbeitsweise einer erfindungsgemäßen Vorrichtung erläutert. Wird die Schutzkappe 18 von dem Betätigungsmittel 14 entfernt, so ändert sich der Schaltzustand des Schutzkappenschalters 25. Die Änderung des Schaltzustandes des Schutzkappenschalters 25 wird als Signal in der Auswerteeinheit und der Zähleinrichtung 21 erfaßt. Das Anzeigelement 22 wird aktiviert. Auf dem LCD-Bildschirm 23 wird dann ein Zählsignal, beispielsweise die Anzahl der bereits erfolgten Betätigungen des Betätigungselements 14 angezeigt. Soweit keine Betätigung des Betätigungselements 14 erfolgt, wird nach einer bestimmten vorgegebenen Zeit - beispielsweise einer Zeit von ca. 30 Sekunden bis 1 Minute - die Anzeige wieder ausgeschaltet.

Ein erneutes Anschalten der Anzeige kann dadurch erfolgen, daß das Betätigungselement 14 leicht betätigt wird und der Leerwegschalter 24 dadurch schaltet. Auch dies führt wieder zu einer Aktivierung des Bildschirmes, zumindest für eine vorgegebene Zeitspanne. Bei einer Betätigung des Betätigungselements 14, die lediglich den Leerwegschalter 24 aktiviert, erfolgt, noch kein Austrag von Medium aus der Austragöffnung 19. Nimmt der Benutzer eine Betätigung des Betätigungselements 14 vor, bei der Medium aus der Austragöffnung 19 ausgetragen wird, wozu das in diesem Bereich als Applikator ausgebildete Betätigungselement mit der Austragöffnung 19 am Applikationsort, beispielsweise in die Nase oder in den Mund eingeführt wird, so wird das Betätigungselement 14 solange relativ zu der Halteschraube 31 bewegt, bis das Schaltelement 26 in Anlage mit der Schaltfläche 27 gewählt. Dadurch wird ein elektrisches Zählsignal erzeugt, das der Zähleinrichtung 21 zugeführt wird. Aufgrund des Zählsignales weiß nun die Zähleinrichtung, daß eine weitere Betätigung des Spenders 11 erfolgt ist. Eine entsprechende Veränderung aufgrund des ermittelten Zählwerts, der sich nun geändert hat, wird ab nun angezeigt. Die Anzeige erlischt entweder nach Ablauf einer vorgegebenen Zeit beispielsweise wiederum einer Zeitspanne (Zeitintervall) von 30 Sekunden bis 1 Minute - oder aber dann, wenn die Schutzkappe 18 wieder auf das Betätigungselement 14 aufgesetzt wird und sich daher der Schaltzustand des Schutzkappenschalters 25 wieder ändert. Es kann aber auch vorgesehen sein, daß ein gewisser Nachlauf nach dem Aufsetzen der Schutzkappe 18 vorgesehen ist. Der Nachlauf bewirkt, daß die Anzeige des LCD-Anzeigeelements 23 noch für eine vorgegebene Zeit nach dem Aufsetzen der Schutzkappe 18 angezeigt wird. Hierbei kann es sich beispielsweise um eine Zeitspanne im Bereich von unterhalb 1 Minute, beispielsweise 10 bis 30 Sekunden handeln. Die einzelnen Zeitintervalle für die Abschaltung (Übergang der Anzeige in den "Nicht-Aktiviert"-Zustand) können dabei auch parallel zueinander laufen, wobei sowohl vorgesehen sein kann, daß das tatsächliche Abschalten mit dem Ablauf des zuletzt endenden Intervalls erfolgt, als auch, daß das Abschalten mit dem Ablauf des zuerst endenden Intervalls erfolgt.

## Patentansprüche

1. Vorrichtung zum Erfassen der Betätigung eines Spenders, insbesondere eines Spenders für feste oder flüssige vorzugsweise wenigstens einen pharmazeutischen Wirkstoff enthaltende Medien, wobei in einem Medienbehälter austragbares Medium bevorratet ist, mit einem Austragförderer, dessen Betätigung den Austrag von Medium zur Folge hat, wobei der Austragförderer durch eine Relativbewegung zwischen einem Betätigungselement und dem Medienbehälter betätigt wird, wobei mit einem Anzeigeelement (22) im Betätigungselement (14) eine Erfassungseinrichtung (20) zur Erfassung erfolgter Betätigungen angeordnet ist, die ein elektrisches Zählsignal zur Erfassung erfolgter Betätigungen erzeugt, **dadurch gekennzeichnet, dass** eine Schutzkappe (18) zum Schutz des Spenders bei Nicht-Gebrauch vorgesehen ist und eine Schutzkappen-Erfassungseinrichtung (25) vorgesehen ist, die das Entfernen der Schutzkappe (18) von dem Spender (11) erfasst und das Anzeigeelement (22) aktiviert.

2. Vorrichtung nach Anspruch 1, **dadurch gekennzeichnet, daß** das elektrische Zählsignal in einer elektrischen Zähleinrichtung (21) verarbeitet wird, wobei die elektrische Zähleinrichtung (21) insbesondere im Betätigungselement (14) angeordnet ist.

3. Vorrichtung nach Anspruch 2, **dadurch gekennzeichnet, daß** in dem Betätigungselement (14) ein Anzeigelement (22) zur Anzeige eines in der Zähleinrichtung (21) in Abhängigkeit der Anzahl erfolgter Zählsignale ermittelten Zählwertes angeordnet ist, wobei das Anzeigeelement als LCD-Anzeige (23) ausgebildet ist.

4. Vorrichtung nach Anspruch 3, **dadurch gekennzeichnet, daß** Anzeigeelement (22) und elektrische Zähleinrichtung (21) als zusammenhängendes Bauteil ausgebildet sind.

5. Vorrichtung nach Anspruch 4, **dadurch gekennzeichnet, daß** die Zähleinrichtung (21) in die Steuereinheit der LCD-Anzeige (23) integriert ist.

6. Vorrichtung nach einem der vorhergehenden Ansprüche, **dadurch gekennzeichnet, daß** in dem Betätigungselement (14) ein elektrooptisches Anzeigeelement (22) angeordnet ist.

7. Vorrichtung nach Anspruch 6, **dadurch gekennzeichnet, daß** das Anzeigeelement (22) als LCD-Anzeige (23) ausgebildet ist.

8. Vorrichtung nach Anspruch 6 oder 7, **dadurch gekennzeichnet, daß** das Anzeigeelement (22) eine Auswerteeinheit aufweist, wobei der Auswerteeinheit Signale eines Sensors zugeführt werden und wobei die Anzeige des Anzeigeelements (22) in Abhängigkeit der Sensorsignale bestimmt wird.

9. Vorrichtung nach einem der Ansprüche 6 bis 8, **dadurch gekennzeichnet, daß** in dem Betätigungselement (14) ein elektrischer Energiespeicher zur Energieversorgung des Anzeigeelements (22) und ggf. der Auswerteeinheit angeordnet ist.

10. Vorrichtung nach einem der Ansprüche 3 bis 9, **dadurch gekennzeichnet, daß** das Anzeigeelement (22) lediglich für eine bestimmte Anzeigedauer aktiviert ist, wobei die Anzeigedauer vorzugsweise auf eine vorgegebene Zeitspanne nach dem letzten die angezeigte Information verändernden Signal begrenzt ist, und wobei vorzugsweise die Aktivierung des Anzeigeelements (22) durch eine nicht zum Medienaustrag führende Betätigung des Betätigungselements (14) erfolgt.

11. Vorrichtung nach Anspruch 10, **dadurch gekennzeichnet, daß** die Schutzkappen-Erfassungseinrichtung (25) das Anordnen der Schutzkappe (18) auf dem Spender erfasst und das Anzeigeelement (22) deaktiviert.

12. Vorrichtung nach einem der vorhergehenden Ansprüche, **dadurch gekennzeichnet, daß** das Betätigungselement (14) als einstückiges, die Austragöffnung (13) des Spenders (11) beinhaltendes Bauteil ausgebildet ist, das insbesondere als am Verabreichungsort anzusetzender Applikator, insbesondere als nasaler oder buccaler Applikator ausgebildet ist.

## Claims

1. Device for detecting the operation of a dispenser, particularly a dispenser for solid or liquid media, preferably containing at least one pharmaceutical substance, a medium container storing dischargeable medium, having a discharge means, whose operation leads to the discharge of the medium, the discharge means being operated by a relative movement between an operating element and the medium container, where with a display element (22) in the operating element (14) is provided a detecting device (20) for detecting operations which have taken place producing an electric counting signal for detecting operations which have taken place, **characterized in that** there is a protective cap (18) for protecting the dispenser when not in use and a protective cap detecting device (25) is provided, which detects the removal of the protective cap (18) from the dispenser (11) and activates the display element (22).

2. Device according to claim 1, **characterized in that** the electric counting signal is processed in an electric counting mechanism (21), which is in particular located in the operating element (14).

3. Device according to claim 2, **characterized in that** in the operating element (14) is provided a display element (22) for displaying a numerical value determined in the counting device (21) as a function of the number of counting signals which have occurred, the display element being constructed as a LCD display (23).

4. Device according to claim 3, **characterized in that** the display element (22) and electric counting mechanism (21) are constructed as a continuous component.

5. Device according to claim 4, **characterized in that** the counting mechanism (21) is integrated into the control unit of the LCD display (23).

6. Device according to one of the preceding claims, **characterized in that** an electrooptical display element (22) is located in the operating element (14).

7. Device according to claim 6, **characterized in that** the display element (22) is constructed as a LCD display (23).

8. Device according to claim 6 or 7, **characterized in that** the display element (22) has an evaluating unit to which are supplied signals of a sensor and the display of the display element (22) is determined as a function of the sensor signals.

9. Device according to one of the claims 6 to 8, **characterized in that** the operating element (14) contains an electric energy storage means for the energy supply of the display element (22) and optionally the evaluating unit.

10. Device according to one of the claims 3 to 9, **characterized in that** the display element (22) is only activated for a given display period, which is preferably limited to a predetermined time interval following the last signal modifying the information displayed and preferably the activation of the display element (22) takes place by an operation of the operating element (14) not leading to medium discharge.

11. Device according to claim 10, **characterized in that** the protective cap detecting device (25) detects the placing of the protective cap (18) on the dispenser and deactivates the display element (22).

12. Device according to one of the preceding claims, **characterized in that** the operating element (14) is constructed as a one-piece component incorporating the discharge opening (13) of the dispenser (11) and which is in particular constructed as an applicator to be fitted at the administration location, particularly as a nasal or buccal applicator.

## Revendications

1. Dispositif de détection d'actionnement d'un distributeur, notamment d'un distributeur pour substances solides ou liquides contenant préférablement au moins un agent pharmaceutique, de la substance à débiter étant stockée dans un récipient à substances, avec un convoyeur de décharge, dont l'actionnement produit le débit de substance, le convoyeur de décharge étant actionné par un mouvement relatif entre un élément d'actionnement et le récipient à substances, par un élément d'affichage (22) étant disposé dans l'élément d'actionnement (14) un moyen de détection (20) pour la détection du nombre d'actionnements effectués, moyen qui génère un signal électrique de comptage pour la détection du nombre d'actionnements effectués, **caractérisé en ce qu'**on prévoit un couvercle protecteur (18) pour la protection du distributeur pendant le non-usage et qu'on prévoit un moyen de détection de couvercle protecteur (25) qui détecte l'éloignement du couvercle protecteur (18) du distributeur (11) et qui active l'élément d'affichage (22).

2. Dispositif d'après la revendication 1, **caractérisé en ce que** le signal électrique de comptage est traité dans un moyen électrique de comptage (21), le moyen électrique de comptage (21) étant disposé notamment dans l'élément d'actionnement (14).

3. Dispositif d'après la revendication 2, **caractérisé en ce que** dans l'élément d'actionnement (14) est disposé un élément d'affichage (22) pour la visualisation d'une valeur numérique déterminée dans le moyen de comptage (21) en fonction du nombre de signaux de comptage qui ont eu lieu, l'élément d'affichage étant réalisé en tant qu'afficheur LCD (23).

4. Dispositif d'après la revendication 3, **caractérisé en ce que** l'élément d'affichage (22) et le moyen électrique de comptage (21) sont réalisés en tant qu'élément constructif connexe.

5. Dispositif d'après la revendication 4, **caractérisé en ce que** le moyen de comptage (21) est intégré dans l'unité de commande de l'afficheur LCD (23).

6. Dispositif d'après une des revendications précédentes, **caractérisé en ce qu'**un élément d'affichage électro-optique (22) est disposé dans l'élément d'actionnement (14).

7. Dispositif d'après la revendication 6, **caractérisé en ce que** l'élément d'affichage (22) est réalisé en tant qu'afficheur LCD (23).

8. Dispositif d'après la revendication 6 ou 7, **caractérisé en ce que** l'élément d'affichage (22) présente une unité d'évaluation, des signaux d'un détecteur étant amenés à l'unité d'évaluation, l'affichage de l'élément d'affichage (22) étant déterminé en fonction des signaux de détecteur.

9. Dispositif d'après une des revendications de 6 à 8, **caractérisé en ce que** dans l'élément d'actionnement (14) est disposé un accumulateur d'énergie électrique pour l'alimentation en énergie de l'élément d'affichage (22) et éventuellement de l'unité d'évaluation.

10. Dispositif d'après une des revendications de 3 à 9, **caractérisé en ce que** l'élément d'affichage (22) est activé uniquement pour une certaine durée d'affichage, la durée d'affichage étant préférablement limitée à un intervalle de temps prédéterminé, après le dernier signal qui modifie l'information affichée, et l'activation de l'élément d'affichage (22) étant de préférence faite par un actionnement de l'élément d'actionnement (14) qui ne mène pas à un débit de substance.

11. Dispositif d'après la revendication 10, **caractérisé en ce que** le moyen de détection de couvercle protecteur (25) détecte le placement du couvercle protecteur (18) sur le distributeur et désactive l'élément d'affichage (22).

12. Dispositif d'après une des revendications précédentes, **caractérisé en ce que** l'élément d'actionnement (14) est réalisé en tant que élément constructif en une partie et comprenant l'ouverture de décharge (13) du distributeur (11), élément constructif qui est notamment réalisé en tant qu'applicateur à apposer à l'endroit d'administration, notamment en tant qu'applicateur nasal ou buccal.
